Europäisches Patentamt

European Patent Office

Office européen des brevets

(19)

(11) Publication number: **0 161 765**
**B1**

# EUROPEAN PATENT SPECIFICATION

(12)

(45) Date of publication of patent specification: **05.10.88**

(21) Application number: **85302122.8**

(22) Date of filing: **27.03.85**

(51) Int. Cl.⁴: **A 61 F 2/16,** G 02 B 1/04, G 02 C 7/04

(54) Ultraviolet light absorbing intraocular implants.

(30) Priority: **11.04.84 US 599005**

(43) Date of publication of application: **21.11.85 Bulletin 85/47**

(45) Publication of the grant of the patent: **05.10.88 Bulletin 88/40**

(84) Designated Contracting States: **DE FR GB IT SE**

(56) References cited:
**EP-A-0 127 937**
**US-A-4 079 470**
**US-A-4 418 431**

(73) Proprietor: **Intermedics Intraocular, Inc. 2650 E. Foothill Boulevard Pasadena California (US)**

(72) Inventor: **Sayano, Reizo 209 Casa Grande Avenue Montebello California 90640 (US)** Inventor: **Goldberg, Eugene P. 14601 Village Glen Circle Tampa Florida 33624 (US)**

(74) Representative: **Dealtry, Brian et al Eric Potter & Clarkson 14, Oxford Street Nottingham NG1 5BP (GB)**

## Description

Certain injuries to the eye and certain diseases to the eye (e.g. cataracts) require surgical removal of the eye lens. Removal of the natural lenses of the eye is known as aphakia which must be corrected by the use of a corrective lens in order to restore vision. Generally, intraocular implants are used to correct aphakia and restore vision. These implants may be permanently placed in the anterior or posterior chamber of the eye. Intraocular implants have an optical lens and a haptic for fixation of the lens, by a surgeon, in the anterior or posterior chamber of the eye. However, even with the most suitable of lenses, vision is not as desirable as it should be for the aphakic individual since such lenses do not adequately compensate for certain changes in light transmission which occur in the absence of the natural human crystalline lens. The result is potential damage to the retina due to increased ultraviolet light transmission.

A considerable portion of incident light entering the eye is absorbed and only the unabsorbed or transmitted portion strikes the retina. Natural light encompasses the entire spectrum of wavelengths in the ultraviolet, visible and infrared radiation ranges. Various artificial light sources also contain many wavelengths.

The crystalline lens of the eye preferentially absorbs a substantial portion of ultraviolet radiation. Accordingly, it is desirable that the lenses of intraocular implants for aphakic individuals, absorb at least 90% of light in the 300 to about 380 nm range but transmit most of the light in the visible spectrum. In addition, intraocular implant lenses are preferably made of a thermoplastic polymer and optically clear, inert to the eye, biocompatible and have a specific gravity of less than about 1.7.

Polymethylmethacrylate sometimes referred to as "PMMA", and various copolymers thereof, have the desired properties discussed above and have been used to make intraocular lenses as well as haptics. PMMA has physical properties which permit it to be formed into intraocular optic lenses that are relatively thin in cross section and, because of PMMA's relatively low specific gravity, about 1.4 or lower, such lenses are relatively comfortable in the eye.

In U.S. Patent No. 4,418,431 is described a biocompatible, stable, chemically inert intraocular implant consisting of an optic lens and a haptic for fixation of said lens in the posterior or anterior chamber of the eye, said optic lens consisting of an optical quality solid thermoplastic polymer. In one embodiment described therein the thermoplastic polymer referred to is polymethylmethacrylate, which has a specific gravity of less than 1.7.

However, PMMA, and copolymers thereof have a serious disadvantage in that they are substantially transparent to ultraviolet radiation which, if transmitted to the retina, can cause eye injury. To avoid this disadvantage intraocular lenses have been fabricated from glass which can absorb ultraviolet radiation. However, compared to PMMA which is relatively easy to machine, glass lenses of relatively thin cross sections are much more difficult to produce. Furthermore since glass lenses have a specific gravity of 2.5 or higher such lenses are relatively heavy and therefore mitigate against the use of such lenses in aphakic individuals.

To overcome the disadvantage of PMMA lenses to ultraviolet radiation transmission, natural and synthetic crystals have also been used to construct intraocular lenses. U.S. Patent No. 4,079,470 discloses a chemically durable optical implant lens formed from a low density natural or synthetic crystal, such as Corundium, Sapphire, Ruby, Sircon, Strontium, Diamond or Anatase. Because of the ability of these materials to absorb ultraviolet radiation such crystals provide an advantage over lenses made from PMMA. However, as with glass, it is more difficult to produce intraocular lenses which have relatively thin cross sections from these crystals. In general, lenses made from such crystals must be considerably thicker than lenses made from PMMA. Crystal lenses like glass lenses, are relatively heavy due to their high specific gravity, about 3.5 or higher, and larger size. Consequently, such crystal lenses are also heavier than the natural lenses of the human eye, and may also impose an undesirable strain to the eye. For these and other reasons, intraocular lenses made from PMMA, tend to be preferred over synthetic or natural crystals and glass lenses.

There is a need for an intraocular implant having an optical lens which is nontoxic, biocompatible and which absorbs a high percentage of ultraviolet light and does not contain leachable or potentially harmful ultraviolet light absorbing additives.

There is also a need for an intraocular optical lens material made of a thermoplastic polymer which is strong, ductile and easily machined or molded into thin sections and having a specific gravity less than about 1.7 and preferably about 1.4 or lower, which is chemically inert and stable, is biocompatible, nonleachable by fluids of the eye, is optically clear and absorbs a major portion of harmful ultraviolet light.

The invention provides a biocompatible, stable, chemically inert intraocular implant comprising an optic lens and a haptic for fixation of said lens in the posterior or anterior chamber of the eye, said optic lens comprising an optical quality solid thermoplastic polymer having a specific gravity of less than 1.7, wherein said polymer has uniformly distributed therein an ultraviolet light absorber in an amount effective to absorb at least 90% of the light in the 300 to 380 nm range but not substantially reducing the amount of light in the visible spectrum, said ultraviolet light absorber being 2-(hydroxy lower alkylphenyl) benzotriazole which, optionally, may be halogen substituted in one or more of the 4, 5, 6 or 7 positions, said benzotriazole being substantially non-toxic and non-leachable in the eye.

The haptics of an intraocular implant in accordance with the invention may have a variety of shapes and can be made of a variety of thermoplastic polymers which are biocompatible, chemically inert, light weight (i.e. have a specific gravity of less than 1.7) strong, tough and flexible and are inert to the fluids in the eye. Such haptics may be made of polypropylene, aromatic polycarbonates, aromatic polyesters, aromatic polyimides, aromatic polyethersulfones, etc.

In an intraocular implant in accordance with the invention the lenses are formulated so that the ultraviolet radiation absorbing substance is non-leachable or nonextractable from the thermoplastic polymer in the presence of eye fluids, and the substance is effective at low concentrations. The intraocular lenses are formulated so that the amount of ultraviolet radiation absorbing substance is small enough so the lens is transparent to most visible radiation but great enough to render the lens absorbent to at least 90% of the ultraviolet radiation of sunlight in the 300 to 380 nm range. The ultraviolet radiation absorbing substance is 2-(hydroxy, lower alkylphenyl) benzotriazole which, optionally, may be halogen (e.g. chlorine) substituted in one or more of the 4, 5, 6 or 7 positions.

Desirably, of course, an intraocular lens of the present invention will have optically finished front and back surfaces and a shape and size approximating the human lens.

The solid thermoplastic polymer may be polymethylmethacrylate, and copolymers thereof with monomers such as methylacrylate, hydroxyethylmethacrylate, ethyl acrylate, butyl acrylate, mixtures thereof, and the like. The particular monomer, or combination of monomers, as well as other additives, such as cross-linking agents and polymerization catalysts, used to form the various polymeric systems are known in the art. Other thermoplastic polymers well suited to be used in both the lens and haptic include aromatic polycarbonates such as Lexan available from General Electric; polysulfones such as Udel P-1700 available from Union Carbide; and polyetherimides such as Ultem available from General Electric.

The ultraviolet light absorbing compounds useful in the present invention are stable, inert, biocompatible, water insoluble, non-leachable and strongly ultraviolet light absorbing compounds based on the benzotriazole structure and include 2 - (3',5' - di - tertiarybutyl - 2' - hydroxy pheny) - 5 - chlorobenzotriazole, 2 - (2' - hydroxy - 5'methylphenyl)benzotriazole and 2 - (3' - tertiarybutyl - 5' - methyl - 2' - hydroxy) - 5 - chlorobenzotriazole.

In the formulation and production of the lenses of this invention, the amount of the ultraviolet radiation absorber will be sufficient to absorb at least 90% of the ultraviolet radiation of sunlight in the 300—380 nm range but will not prevent the lens from being transparent to a substantial part of the visible spectrum.

The lenses of this invention may be easily produced using standard procedures such as lathe cutting, injection molding, compression molding and casting.

The ultraviolet light absorbing compound may be uniformly distributed through the thermoplastic polymer by the polymerization of the monomers in the presence of the ultraviolet radiation absorbing compound. Alternatively, the ultraviolet light absorbing compound may be compounded with the thermoplastic polymer prior to extrusion or molding. The amount of the absorbing compound is preferably from about 0.01 to about 5 parts by weight per 100 parts by weight of the polymer. The ultraviolet light-absorbing compounds used in this invention are inert and do not adversely affect the polymerization of the monomers used to produce the thermoplastic polymers, or their processing by molding or extrusion.

Figure 1 is an illustration in plan view of an intraocular implant for implantation in the anterior chamber of the eye.

Figure 2 is a side view of the intraocular implant of Figure 1.

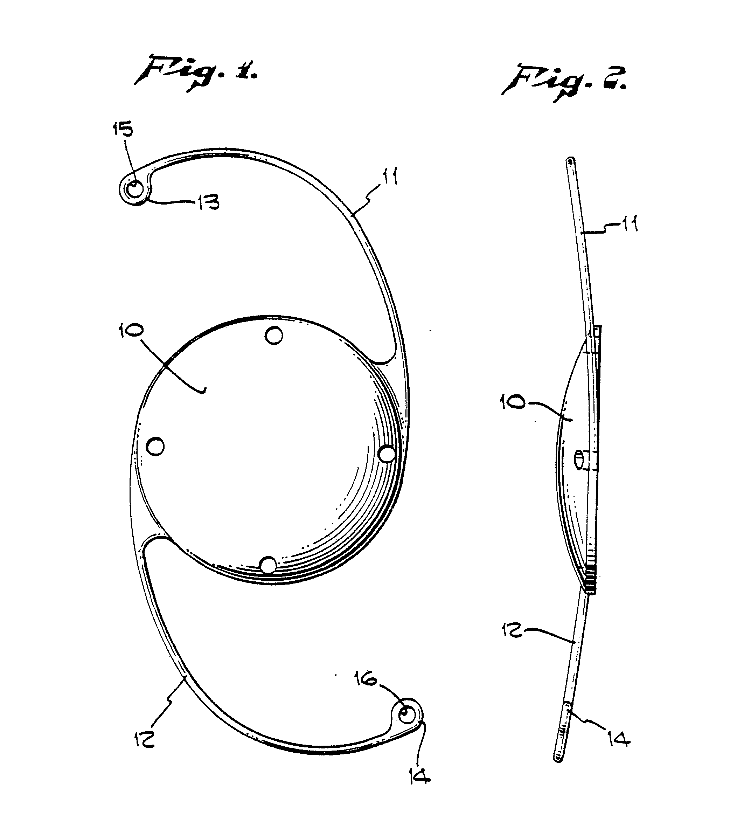

An intraocular implant embodying the present invention as shown in Figures 1 and 2 has a generally circular optic lens 10 which is made of a thermoplastic polymer having uniformly distributed therethrough an ultraviolet light absorbing amount of 2-(hydroxy-lower alkylphenyl) benzotriazole which may be halogen substituted in the 4, 5, 6 or 7 positions. The amount of benzotriazole distributed throughout the polymer is preferably from 0.01 to 5 parts by weight of the thermoplastic polymer. ·

The ultraviolet light absorbing substance may be mixed with the monomers and then the monomers polymerized or by compounding with the thermoplastic polymer prior to extrusion or molding.

In addition to the optic lens 10, the intraocular implant of the present invention also has haptics connected to the optic lens for positioning and fixing the implant in either the anterior or posterior chamber of the eye. In the embodiment of this invention shown in Figures 1 and 2, haptic 11 and haptic 12 are resilient so that they can be compressed when being placed in the eye but will spring out when the implant is in the correct position so that positioning element 13 of haptic 11 and positioning element 14 of haptic 12 will contact and be seated in the groove of the anterior chamber of the eye.

Aperture 15 and aperture 16 are provided for grasping with forceps.

The haptics may be made of any thermoplastic polymer which is strong and lightweight, chemically inert and biocompatible. The haptic may be made of the same material as the lens, e.g., polysulfone, polycarbonate, etc., or may be different, e.g. a PMMA optic with polypropylene haptic.

Example 1

Three fine dispersions of 200 micro-grams of each of the following ultraviolet light absorbing compounds in a 0.9% saline solution were made: 2 - (3',5' - ditertiarybutyl - 2' - hydroxy phenyl) benzotriazole (hereinafter DHP), 2 - (2' - hydroxy - 5' - methylphenyl) benzotriazole (hereinafter HMP), and 2 - (3' - tertiary butyl - 5' - methyl - 2' - hydroxy phenyl) - 5 - chloro-benzo - triazole (hereinafter TMP). Each of the three saline solutions were injected, respectively, into the anterior chamber of the eyes of three rabbits. This amount is the equivalent of 1 weight % of ultraviolet light absorbing compound in a 20 mg. lens.

No adverse reactions were noted. The animals were followed for one week and eyes were normal and tissue histology was normal. This test demonstrates the non-toxic biocompatible properties of the ultraviolet light absorbing compounds.

Example 2

100 parts by weight of polysulfone (Udel P-1700) pellets were mixed with 8 parts by weight of TMP powder. The mixture was extruded into a rod at 550°F to 600°F (about 288°C to 316°C) and then cut into discs of about 1.0 mm thickness. The discs absorbed over 95% of the ultraviolet light in the 300 to 400 nm region and had a sharp cut-off of absorption between 400 and 420 nm.

Example 3

100 parts by weight of polysulfone (Udel P-1700) pellets were mixed with 10 parts by weight of DHP powder. This mixture was compression molded into a 1 mm thick sheet which absorbed over 95% of the ultraviolet light in about the 300 to 400 nm region and had a sharp cut-off of absorption in the 400 to 420 nm region (there was 5% transmission at 403 nm and 70% at 420 nm).

Example 4

A sheet, 2.95 mm thick, was made having 100 parts by weight of PMMA and 10 parts by weight of DHP. The sheet absorbed more than 95% of ultraviolet light in the 300—400 nm region and had a sharp cut-off of absorption in about the 405 to 430 nm range (there was less than 5% transmission at 405 nm and about 90% transmission at 430 nm).

Example 5

A sheet, 1/8 inch (3.18 mm) thick, was made having 100 parts by weight of PMMA and 10 parts by weight of HMP. This sheet absorbed over 95% of the ultraviolet light between 300 and 400 nm with a sharp cut-off of absorption at about 400 nm (there was about 5% transmission at 400 and over 90% transmission at about 425 nm).

Example 6

The PMMA containing HMP material used in Example 5 was used in this example. Four samples of the material were extracted in four (4) different media for one (1) hour at 121°C.
   1. Sodium chloride
   2. Ethanol in sodium chloride
   3. Polyethylene glycol
   4. Cottonseed oil

Two rabbits were used for each extract. Exactly 0.2 ml of test material extract was injected intracutaneously in ten (10) sites on the right side of each animal and ten (10) injections of 0.2 ml of extracting medium were placed into the left side of each animal. The degree of erythema and edema of the two sides were compared 1, 2 and 3 days after the injection to determine the degree of tissue reaction.

There were no significant signs of erythema nor edema due to the intracutaneous injection of extraction of the PMMA material as compared to injections of the extraction mediums. Therefore, an extract of PMMA material does not result in erythema or edema 72 hours after intracutaneous injection. This test demonstrates the non-toxic and non-leachable properties of the ultraviolet light absorbing additive.

Example 7

Four (4) grams of material having the same composition as used in Example 6 were cut into 11 pieces approximately 1.016 cm square and 0.3 cm thick (37.07 cm² total surface area for each 4 grams). The materials were cleaned and stearilized. The 4 grams were then incubated at 90°C in 20 ml of saline (1 gr for each 5 ml of saline or 1 cm² surface area for each 0.55 ml of saline). After 1.5 weeks of 90°C, the material was removed and the ultraviolet absorbance of the extract measured. The concentration of the absorber in the extract was determined from the absorbance.

The absorbance of pure saline subtracted from the absorbance of the extract of the ultraviolet absorbing PMMA, was always less than 0.004 between wavelengths of 300 and 400 nm. Since the ultraviolet absorber strongly absorbs ultraviolet radiation and is stable above 140°C, it would have been detected by this method. Therefore, there was no significant leaching of ultraviolet absorber, since there was negligible absorbance of the ultraviolet filtering PMMA extract.

Example 8

Intraocular implants of PMMA containing 0.1 weight percent of HMP were implanted in the anterior chamber of rabbits. No toxic or adverse behavior was observed after one year. There was also one explanted lens which showed no change in ultraviolet light absorbing properties.

**Claims**

1. A biocompatible, stable, chemically inert intraocular implant comprising an optic lens and a haptic for fixation of said lens in the posterior or anterior chamber of the eye, said optic lens comprising an optical quality solid thermoplastic polymer having a specific gravity of less than 1.7, characterised in that said polymer has uniformly distributed therein an ultraviolet light absorber in an amount effective to absorb at least 90% of the light in the 300 to 380 nm range but not substantially reducing the amount of light in the visible spectrum, said ultraviolet light absorber being 2-(hydroxy lower alkylphenyl) benzotriazole which, optionally, may be halogen substituted in one or more of 4, 5, 6 or 7 positions, said benzotriazole being substantially non-toxic and non-leachable in the eye.

2. An intraocular implant according to Claim 1 characterised in that the solid thermoplastic polymer is polymethylmethacrylate or copolymers thereof.

3. An intraocular implant according to Claim 1 characterised in that the solid thermoplastic polymer is aromatic polycarbonate.

4. An intraocular implant according to Claim 1 characterised in that the solid thermoplastic polymer is aromatic polysulfone.

5. An intraocular implant according to Claim 1 characterised in that the solid thermoplastic polymer is aromatic polyetherimide.

6. An intraocular implant according to Claim 1 characterised in that the amount of ultraviolet light absorber is between 0.01 to 5 parts by weight per 100 parts by weight of the solid thermoplastic polymer.

7. An intraocular implant according to Claim 1 characterised in that the halogen is chlorine.

8. An intraocular implant according to Claim 1 characterised in that the halogen is in the 5 position.

9. An intraocular implant according to Claim 1 characterised in that said benzotriazole is unsubstituted in the 4, 5, 6 and 7 positions.

## Patentansprüche

1. Bioverträgliches, stabiles, chemisch inertes Intraokularimplantat aus einer optischen Linse und einem Mittel zur Fixierung der Linse in der hinteren oder vorderen Kammer des Auges, wobei die optische Linse aus einem festen, thermoplastischen, optische Qualität aufweisenden Polymerisat eines spezifischen Gewichts von weniger als 1,7 besteht, dadurch gekennzeichnet, daß in dem Polymerisat ein UV-Lichtabsorptionsmittel in einer Menge, die zwar mindestens 90% des Lichts im Bereich 300—380 nm wirksam absorbiert, jedoch die Lichtmenge im sichtbaren Spektralbereich praktisch nicht vermindert, gleichmäßig verteilt enthält, wobei das UV-Lichtabsorptionsmittel aus einem gegebenenfalls ein- oder mehrfach in 4-, 5-, 6- bzw. 7-Stellungen halogensubstituierten 2-(Hydroxyniedrigalkylphenyl)-benzotriazol, das praktisch nicht-toxisch und nicht in das Auge auslaugbar ist, besteht.

2. Intraokularimplantat nach Anspruch 1, dadurch gekennzeichnet, daß das feste thermoplastische Polymerisat aus Polymethylmethacrylat oder Mischpolymerisaten desselben besteht.

3. Intraokularimplantat nach Anspruch 1, dadurch gekennzeichnet, daß das feste thermoplastische Polymerisat aus einem aromatischen Polycarbonat besteht.

4. Intraokularimplantat nach Anspruch 1, dadurch gekennzeichnet, daß das feste thermoplastische Polymerisat aus einem aromatischen Polysulfon besteht.

5. Intraokularimplantat nach Anspruch 1, dadurch gekennzeichnet, daß das feste thermoplastische Polymerisat aus einem aromatischen Polyetherimid besteht.

6. Intraokularimplantat nach Anspruch 1, dadurch gekennzeichnet, daß die Menge an UV-Lichtabsorptionsmittel 0,01—5 Gew.-Teil(e) pro 100 Gew.-Teile des festen thermoplastischen Polymerisats beträgt.

7. Intraokularimplantat nach Anspruch 1, dadurch gekennzeichnet, daß das Halogen aus Chlor besteht.

8. Intraokularimplantat nach Anspruch 1, dadurch gekennzeichnet, daß sich das Halogen in 5-Stellung befindet.

9. Intraokularimplantat nach Anspruch 1, dadurch gekennzeichnet, daß das Benzotriazol in den 4-, 5-, 6- und 7-Stellungen unsubstituiert ist.

## Revendications

1. Implant intraoculaire biocompatible, stable, chimiquement inerte, comprenant une lentille optique et une attache pour fixer ladite lentille dans la chambre postérieure ou antérieure de l'oeil, ladite lentille optique comprenant un polymère solide thermoplastique de qualité optique ayant une gravité spécifique inférieure à 1,7, caractérisé en ce que ledit polymère contient, à l'état uniformément réparti, un absorbant de lumière ultra-violette en quantité effective pour absorber au moins 90% de la lumière dans la gamme de 300 à 380 nm, mais sans réduire appréciablement la quantité de lumière dans le spectre visible, ledit absorbant de lumière ultra-violette étant de 2 - (hydroxy - infra - alkylphényle) benzotriazole pouvant être optionnellement substitué avec un halogène en une ou plusieurs des positions 4, 5, 6 ou 7, ledit benzotriazole étant pratiquement non-toxique et insoluble dans l'oeil.

2. Implant intraoculaire selon la revendication 1, caractérisé en ce que le polymère solide thermoplastique est le polyméthylméthacrylate ou des copolymères de celui-ci.

3. Implant intraoculaire selon la revendication 1, caractérisé en ce que le polymère solide thermoplastique est un polycarbonate aromatique.

4. Implant intraoculaire selon la revendication 1, caractérisé en ce que le polymère solide thermoplastique est un polysulfone aromatique.

5. Implant intraoculaire selon la revendication 1, caractérisé en ce que le polymère solide thermoplastique est un polyétherimide aromatique.

6. Implant intraoculaire selon la revendication 1, caractérisé en ce que la quantité d'absorbant de la lumière ultra-violette est entre 0,01 et 5 parties en poids pour 100 parties en poids du polymère solide thermoplastique.

7. Implant intraoculaire selon la revendication 1, caractérisé en ce que l'halogène est le chlore.

8. Implant intraoculaire selon la revendication 1, caractérisé en ce que l'halogène est en position 5.

9. Implant intraoculaire selon la revendication 1, caractérisé en ce que ledit benzotriazole est insubstitué dans les positions 4, 5, 6 et 7.

0 161 765

Fig. 1.

Fig. 2.

15

11

13

10

12

16

12

14

14

11

10

12

1